# EUROPEAN PATENT SPECIFICATION

(11) **EP 1 373 825 B1**
(45) Date of publication and mention of the grant of the patent: **21.02.2007**
(21) Application number: 00981534.1
(22) Date of filing: 15.12.2000
(51) Int. Cl.: G01B 3/10

(54) **PAEDIATRIC ELECTRONIC DEVICE FOR MEASURING LENGTH**
PÄDIATRISCHE ELEKTRONIK-VORRICHTUNG ZUR LÄNGENMESSUNG
DISPOSITIF ELECTRONIQUE PEDIATRIQUE DE MESURE DE LONGUEUR

(30) Priority: 20.12.1999 ZA 9907769
(43) Date of publication of application: 02.01.2004
(73) Proprietor: Naidoo, Maurice Kelvin, Sandton, J.H.B. 2196 (ZA)
(72) Inventor: Naidoo, Maurice Kelvin, Sandton, J.H.B. 2196 (ZA)
(74) Representative: 't Jong, Bastiaan Jacobus
(86) International application number: PCT/IB2000/001880
(87) International publication number: WO 2001/046642

(56) References cited:
- EP-A- 0 220 860
- EP-A- 0 357 533
- US-A- 4 718 507
- US-A- 5 477 622
- US-A- 6 094 996
- PATENT ABSTRACTS OF JAPAN vol. 018, no. 135 (P-1705), 7 March 1994 (1994-03-07) & JP 05 322501 A (MITSUTOYO CORP), 7 December 1993 (1993-12-07)
- PATENT ABSTRACTS OF JAPAN vol. 1998, no. 01, 30 January 1998 (1998-01-30) & JP 09 238926 A (SHIMADZU CORP), 16 September 1997 (1997-09-16)
- PATENT ABSTRACTS OF JAPAN vol. 1999, no. 01, 29 January 1999 (1999-01-29) & JP 10 288545 A (HOKUEI KOGYO KK), 27 October 1998 (1998-10-27)

## Description

This invention relates to a paediatric electronic device. More particularly, this invention relates to a paediatric electronic device whereby a person is provided with information relating to a young child. The person may be a doctor or parent and the information may relate to physical measurements, suitable drugs, dosages, medical procedures, equipment, milestones and clothing.

In this specification the term "young child" includes a baby.

Patent Abstracts of Japan vol. 018, no 135 (P-1705) & JP 05 322501 A (MITSUTOYO CORP) relates to an electronic tape measure to determine the volume and weight of a rectangular article.

EP-A-O 220 860 (BROSELOW James B) discloses a measuring tape which has indicia thereon for measuring a patient, the indicia representing increments of a physical treatment or phsysiological value based upon a correlation between the length of a patient and that value.

US-A-4 718 507 (HOWLETT Clyde W et al) discloses an electronic scale and length measurement apparatus.

According to the invention there is provided a paediatric electronic device which includes
a housing;
a length measuring means for measuring the length of a young child;
a memory means for storing data in regard to various weight dependent parameters; and
a display means for displaying the values of the parameters, characterised therein that the parameters are grouped in various groups; and
the device further includes a group selecting means for selecting a desired group of parameters and a processing means for determining the value of various parameters in the selected group in accordance with the length of the young child as measured by the length measuring means.

The parameter groups may be selected from physical measurements, normal values of clinical parameters, settings for ventilatory equipment, drug infusion quantities, normal biochemistry values, normal Values related to x-rays and electrocardiograms, infusion equipment sizes, airway equipment sizes, and resuscitation drug quantities.

The particular values of the various parameters for each length may be stored in a data base or may be computed in accordance with a formula, stored in the memory means.

The memory means may store a length of a young child that has been measured, until reset.

The measuring means may include a length of tape housed in the housing and extractable therefrom, with an electronically operable monitoring means for monitoring the extent to which the tape has been extracted from the housing. A retraction means may also be provided to automatically retract the tape into the housing. Preferably a locking means is provided to lock the measuring tape when it has been extracted to a desired extent.

The memory means may be reset when the measuring tape is extracted from the housing after having been retracted into the housing.

The monitoring means may include an optical sensor responsive to formations on the tape, the number of formations passing past the sensor being counted. The formations may be openings at spaced intervals in the tape.

The group selecting means may include a plurality of buttons which are selectively depressed by the person using the device to select which group of parameters is to be displayed.

The device may include a power supply and a back-up power supply. The device may further have a "sleep" function whereby power consumption is reduced if the device is not operated for a specified time period. Further, a low voltage monitor may be included together with a low voltage warning annunciator.

The display means may include a back-lit LCD display matrix with suitable drivers.

The invention is now described, by way of an example, with reference to the accompanying drawings, in which:-
Figure 1 shows a schematic front perspective view of a paediatric electronic device in accordance with the invention;
Figure 2 shows a bottom view of the device;
Figure 3 shows a perspective view of a holder for the device;
Figure 4 shows a circuit diagram of a monitoring circuit for monitoring the extent to which a tape of the device is extracted; and
Figure 5 shows a schematic block diagram of electronic componentry of the device.

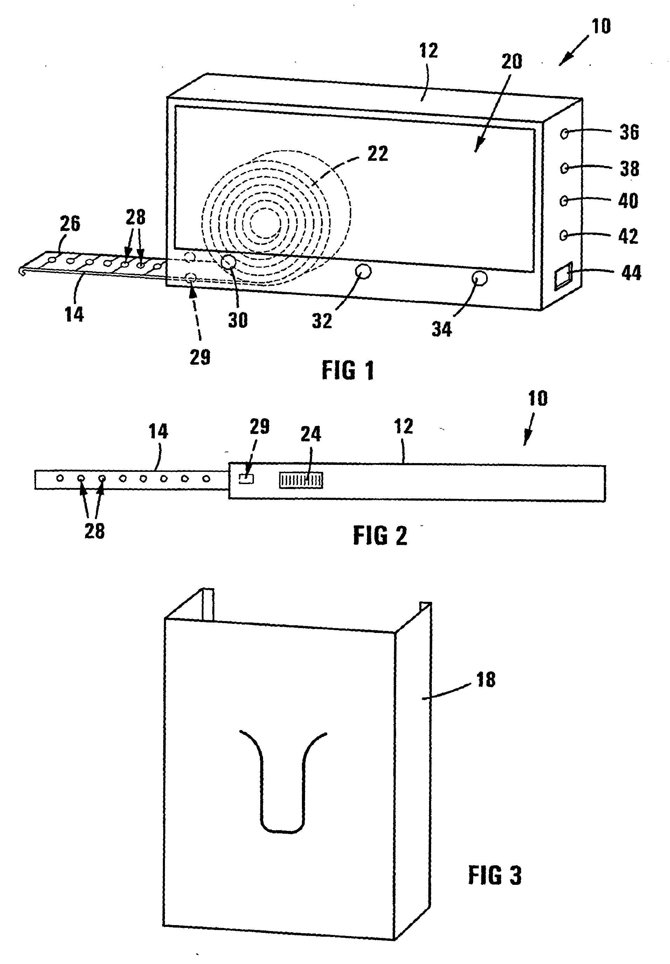

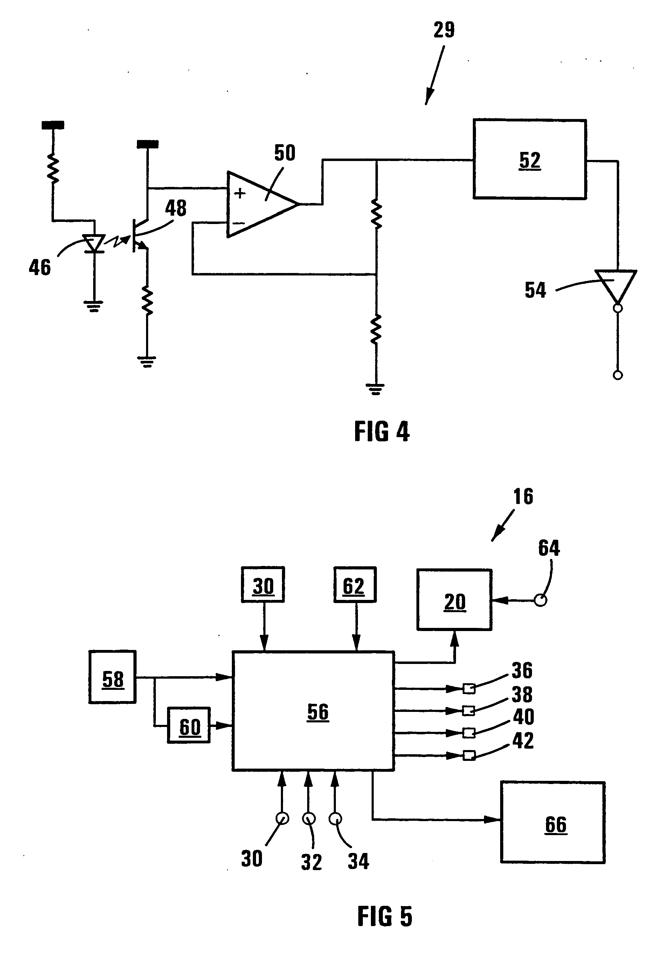

Referring now to Figure 1 of the drawings, a paediatric electronic device in accordance with the invention is designated generally by reference numeral 10. The device 10 has a housing 1 2 in which are housed a flexible metal measuring tape 14 and electronic componentry 16. The electronic componentry will be described hereinbelow with reference to Figure 5. The housing 12 is rectangular and is of a similar size to pagers, such that it can be held in the hand of a user and control buttons operated, and can be carried in a holder, such as the holder 18 shown in Figure 3, on the belt of a user or in a coat pocket.

The housing 12 has mounted therein a back-lit LCD display 20 on which the values of various parameters are displayed, depending on the extent to which the tape 14 is extracted, as will also be described more fully below.

The tape 14 is coiled within the housing 12 on a spool that is spring loaded by means of a coil spring 22 so that the tape 14 may be extracted a desired extent and is automatically retracted. A lock, which is operated frictionally by a slide 24, locks the tape 14 in a desired extracted extent.

The tape 14 has gradations 26 thereon and a number of central spaced apertures 28. The electronic componentry has an optical sensor 29 which senses the passage of the apertures 28, thereby to monitor the extent to which the tape 14 has been extracted.

On the front of the housing 12, below the display 20, there are three control buttons 30, 32 and 34. These control the parameters displayed, as will be described below.

Four differently coloured LED's 36, 38, 40 and 42 are mounted on the right side of the housing 12. The tape 14 is divided into seven sectors and the appropriate LED is energised, depending on the extent to which the tape 14 has been extended, to indicate a range. An "on/off" switch button 44 is also located on the right side of the housing 12.

A battery (not shown) is also housed in the housing 12 to provide power for the electronic componentry. A back-up battery (also not shown) is also provided.

Referring now to Figure 4, the optical sensor 29 is shown. The sensor 29 comprises an LED 46 and phototransistor 48, which drives an op-amp 50. The op-amp 50 is connected to an inverted Schmitt trigger 52 which drives an inverter 54. The LED 46 is positioned on one side of the tape 14 and the phototransistor 48 on the other side, so that when an aperture 28 is aligned therewith, the light from the LED 46 is detected by the phototransistor and the Schmitt trigger 52. operated. The inverter 54 thus provides a series of signals to a processor (described below) as the tape 14 is moved past the sensor 29.

The electronic componentry is now described with reference to Figure 5. The componentry includes a processor 56 which is supplied with power from the battery 58. A low voltage monitor 60 monitors the condition of the battery 58 and the processor 56 responds to a low voltage condition by displaying a suitable message on the display 20, or an LED (not shown). The sensor 29 is connected to the processor 56 and provides it with tape extension signals as described above. A reset signal is supplied from a reset switch 62. The processor 56 is also connected to the range indicating LED's 36, 38, 40 and 42, as are the control buttons 30, 32 and 34. A further press-button switch 64 operates back-lighting of the display 20. Finally, the componentry also includes a memory module 66. The memory module 66 stores any program required for the processor as well as the relevant data required by the processor to display the values of the desired parameters on the display 20 in accordance with the extent to which the tape has been extracted.

The various parameters that are displayed, and illustrative values are now described by listing the various screen displays that are provided in the exemplary device. It is to be noted that the bottom line of each display is for control purposes and the expressions therein are associated with the appropriate control button 30, 32 or 34 aligned therewith.

| FIRST SCREEN: | |
|---|---|
| AGE : | 8 YRS |
| WEIGHT: | 24 KG |
| SELECT OPTION? | |
| NVALU/EQUIP/DRUG | |

| SECOND SCREEN: | |
|---|---|
| NVALU | PHYSICAL |
| RR - | 15 - 26 b/m |
| PR - | 60 - 140/60 - 90 |
| BP - | 97 - 112/57 - 71 |
| T° OR - | 35.6 - 37.6 dc |
| AX - | 35.2 - 37.2 dc |
| RE - | 35.7 - 37.8 dc |
| BIOC/XR | ECG/MAIN |

| THIRD SCREEN: | |
|---|---|
| EQUIP | VENT SET |
| 02 | 1 00 % |
| TV - | 240 - 360 ml/kg |
| IT ≥ | 0.6 SEC |
| PIP - | 20 - 30 cmH2O |
| VR - | 15 - 20 b/m |
| PEEP - | 3-5 cmH2O |
| IV ETC/AIR/MAIN | |

| FOURTH SCREEN | |
|---|---|
| DRUG | INFUSIONS |
| EPI - | 1.4 mg in 100 |
| NOR ml@ - | 5 - 25 ml/hr |
| DOP - | 144 mg in 100 |
| DOB ml@ - | 5 - 20 ml/hr |
| LID - | 288 mg in 100 |
| ml@ - | 10 - 25 ml/hr |
| RESUS1/RES2/MAIN | |

| FIFTH SCREEN: | |
|---|---|
| NVALU | BIOCHEM |
| PH - | 7.35 - 7.45 |
| PaCO2 - | 32 - 48 mmHg |
| PaO2 - | 80 - 105 mmHg |
| Hb - | 13.5 g/dl |
| UREA/Cr - | 27 - 62 µmol/l |
| PHYS/XR ECG/MAIN | |

| SIXTH SCREEN: | |
|---|---|
| NVALU | XR ECG |
| PR | > 0.1 sec |
| QRS DURATION | 0.07 s |
| QRS/P AXIS | 60/0 - 90 |
| CSP ODO SPA | ≤ 3 mm |
| PRE VER | ≤ 5 mm |
| COR DIA | ≥ 13 mm |
| BIOCHM/PHYS/MAIN | |

| SEVENTH SCREEN: | |
|---|---|
| EQUIP | HIV ETC |
| JELCO - | 18 - 20 G |
| B/FLY - | 21 - 22 G |
| NGT - | 14-18F |
| U CAT - | 12F |
| lCD - | 28-32F |
| VENT/AIRWAY/MAIN | |

| EIGHTH SCREEN: | |
|---|---|
| EQUIP | AIR WAY |
| ETT UNCU | 6.0 mm CUFFED |
| T-T cm | 17-18 cm |
| L/SCOPE | 2-3 STR |
| | OR CURVE |
| STYLET | 14 F |
| SUCTION CAT | 10 F |
| VENT/lV ETC/MAIN | |

| NINTH SCREEN: | | |
|---|---|---|
| DRUG | RESUS1 | |
| EPI | 1:10000 | 2.4 ml |
| EPI | 1 :1000 | 4.8 ml |
| ATR | 0.01% | 4.8 ml |
| BIC | 24 ml mEQ | 24 ml |
| LID | 2% | 1 - 2 ml |
| DFIB | | 48 J/96 J |
| INFU/RESUS2/MAIN | | |

| TENTH SCREEN: | | |
|---|---|---|
| DRUG | RESUS2 | |
| CALCl | 1 % | 4.8 ml |
| FUROS | | 20 mg |
| DIAZE | | 2.4 - 7.2 mg |
| PHENO | | 360 - 480 mg |
| D2SW | 25 % | 48 ml |
| LORAZ | | 2.4 mg |
| INFU/RESUS1/MAIN | | |

The abbreviated expressions in the screen displays have the following meanings:-
- AGE -: Age
- AIR -: Airway
- AIRWAY -: Airway
- ATR -: Atropine
- B/FLY -: Butterfly
- BIC -: Bicarbonate
- BIOC -: Biochemistry
- BIOCHM -: Biochemistry
- BP -: Blood pressure
- COR DIA -: Cord diameter
- CDO SPA -: Odontoid space
- CXR -: Chest x-ray
- DFIB -: Defibrillation
- DIAZE -: Diazepam
- DOP -: Dopamine
- DRUG -: Drug (or medication)
- DRUG INFUSIONS -: Drug infusions
- D2SW -: 25% dextrose water
- EPI -: Epinephrine
- EQUIP AIR WAY -: Equipment airway
- EQUIP IV ETC -: Equipment intravenous, et cetera
- EQUIP VENT SET -: Equipment ventilatory settings
- ETT -: Endotracheal tube
- FUROS -: Furosemide
- CALCI -: Calcium
- Hb -: Haemoglobin
- I C D -: Intercostal drain
- INFU -: Infusions
- IT -: Inspiratory time
- IV ETC -: Intravenous et cetera
- JELCO -: Jelco
- LID -: Lidocaine
- LORAZ -: Lorazepam
- MAIN -: Main menu
- mEQ -: Milli equivalents
- N G T -: Nasogastric tube
- NOR ml@ -: ml/h - Norepinephrine (in millilitres) at x millilitres per hour
- NVALU BIOCHEM -: Normal values of biochemistry
- NVALU PHYSICAL -: Normal values of clinical parameters
- NVALU XR ECG -: Normal values related to x-ray and electrocardiogram
- 02 -: Oxygen
- PaCO2 -: Partial pressure of carbon dioxide in arterial blood
- Pa02 -: Partial pressure of oxygen in arterial blood
- PH -: Unit for measurement of acidity of alkalinity
- PHENO -: Phenobarbitone
- PHYS -: Physical
- PEEP -: Peak end expiratory pressure
- PIP -: Peak inspiratory pressure
- PR -: Interval between P and R waves on ECG
- PRE VER -: Prevertebral soft tissue shadow
- QRS -: QRS complex on ECG
- RES2 -: Resuscitation 2
- RESUS1 -: Resuscitation 1
- RESUS2 -: Resuscitation 2
- RE -: Rectal temperature
- RR -: Respiratory rate
- STYLET -: Stylet
- SUCTION CAT -: Suction catheter
- T^{o} OR -: Oral temperature
- AX-: Axillary temperature
- T-T -: Trachea to teeth distance
- TV -: Tidal volume
- U CAT -: Urinary catheter
- UNCU -: Uncuffed
- UREA/Cr -: Urea / creatinine
- VENT -: Ventilation
- XR ECG -: X-ray, electrocardiogram
- VR -: Ventilation rate

The manner in which a user navigates between the screens will now be described:-
If the first screen is displayed and button 30 is pressed,then the second screen is displayed; if button 32 is pressed then the third screen is displayed; and if button 34 is pressed then the fourth screen is displayed.
If the second screen is displayed and button 30 is pressed, then the fifth screen is displayed; if button 32 is pressed then the sixth screen is displayed; and if button 34 is pressed then the first screen is displayed.
If the third screen is displayed and button 30 is pressed, then the seventh screen is displayed; if button 32 is pressed then the eighth screen is displayed; and if button 34 is pressed then the first screen is displayed.
If the fourth screen is displayed and button 30 is pressed, then the ninth screen is displayed; if button 32 is pressed then the tenth screen is displayed; and if button 34 is pressed then the first screen is displayed.
If the fifth screen is displayed and button 30 is pressed, then the second screen is displayed; if button 32 is pressed then the sixth screen is displayed; and if button 34 is pressed then the first screen is displayed.
If the sixth screen is displayed and button 30 is pressed, then the fifth screen is displayed; if button 32 is pressed then the second screen is displayed; and if button 34 is pressed then the first screen is displayed.
If the seventh screen is displayed and button 30 is pressed, then the third screen is displayed; if button 32 is pressed then the eighth screen is displayed; and if button 34 is pressed then the first screen is displayed.
If the eighth screen is displayed and button 30 is pressed, then the third screen is displayed; if button 32 is pressed then the seventh screen is displayed; and if button 34 is pressed then the first screen is displayed.
If the ninth screen is displayed and button 30 is pressed, then the fourth screen is displayed; if button 32 is pressed then the tenth screen is displayed; and if button 34 is pressed then the first screen is displayed.
If the tenth screen is displayed and button 30 is pressed, then the fourth screen is displayed; if button 32 is pressed then the ninth screen is displayed; and if button 34 is pressed then the first screen is displayed.

## Claims

1. A paediatric electronic device (10) which includes
a housing (12);
a length measuring means (14, 29) for measuring the length of a young child;
a memory means (66) for storing data in regard to various weight dependent parameters; and
a display means (20) for displaying the values of the parameters, **characterised** therein that the parameters are grouped in various groups; and
the device further includes a group selecting means (30, 32, 34) for selecting a desired group of parameters and a processing means (56) for determining the value of various parameters in the selected group in accordance with the length of the young child as measured by the length measuring means.

2. The paediatric electronic device as claimed in Claim 1, **characterised** therein that the parameter groups are selected from physical measurements, normal values of clinical parameters, settings for ventilatory equipment, drug infusion quantities, normal biochemistry values, normal values related to x-rays and electrocardiograms, infusion equipment sizes, airway equipment sizes, and resuscitation drug quantities.

3. The paediatric electronic device as claimed in Claim 1 or 2, **characterised** therein that at least some of the particular values of the various parameters for each length are stored in a data base.

4. The paediatric electronic device as claimed in Claim 1 or 2, **characterised** therein that the particular values of some of the various parameters for each length are computed in accordance with a formula, stored in the memory means.

5. The paediatric electronic device as claimed In any one of the preceding claims, **characterised** therein that the memory means stores a length of a young child that has been measured, until reset.

6. The paediatric electronic device as claimed in any one of the preceding claims, **characterised** therein that the measuring means includes a length of tape (14) housed in the housing and extractable therefrom, with an electronically operable monitoring means (29) for monitoring the extent to which the tape has been extracted from the housing.

7. The paediatric electronic device as claimed in Claim 6, **characterised** therein that it includes a retraction means (22) to automatically retract the tape into the housing.

8. The paediatric electronic device as claimed in Claim 6 or 7, **characterised** therein that it includes a locking means (24) to lock the measuring tape when it has been extracted to a desired extent.

9. The paediatric electronic device as claimed in any one of Claims 6 to 8, **characterised** therein that the memory means is reset when the measuring tape Is extracted from the housing after having been retracted into the housing.

10. The paediatric electronic device as claimed in any one of Claims 6 to 9, **characterised** therein that the monitoring means includes an optical sensor (29) responsive to formations on the tape, the number of formations passing past the sensor being counted.

11. The paediatric electronic device as claimed in Claim 10, in which the formations are openings (28) at spaced intervals in the tape.

12. The paediatric electronic device as claimed in any one of the preceding claims, in which the group selecting means includes a plurality of buttons (30, 32, 34) which are selectively depressed by the person using the device to select which group of parameters is to be displayed.

13. The paediatric electronic device as claimed in any one of the preceding claims, **characterised** therein that it includes a power supply (58) and a back-up power supply.

14. The paediatric electronic device as claimed in Claim 13, **characterised** therein that it has a "sleep" function whereby the power consumption is reduced if the device is not operated for a specified time period.

15. The paediatric electronic device as claimed in Claim 13 or 14, **characterised** therein that it includes a low voltage monitor (60) together with a low voltage warning annunciator (20).

16. The paediatric electronic device as claimed in any one of the preceding claims, **characterised** therein that the display means includes a bark-lit LCD display matrix with suitable drivers (20).

## Patentansprüche

1. Pädiatrisches elektronisches Gerät (10), welches umfasst:
ein Gehäuse (12);
eine Längenmessvorrichtung (14, 29) zum Messen der Länge eines Kindes;
eine Speichereinrichtung (66) zum Speichern von Daten bezüglich verschiedener gewichtsabhängiger Parameter; und
ein Anzeigemittel (20) zum Anzeigen der Werte der Parameter,
**dadurch gekennzeichnet, dass** die Parameter in verschiedene Gruppen gruppiert sind; und
dass das Gerät ferner Gruppenwählmittel (30, 32, 34) zur Auswahl einer gewünschten Gruppe von Parametern und eine Verarbeitungseinrichtung (56) aufweist zum Bestimmen des Wertes von verschiedenen Parametern in der ausgewählten Gruppe entsprechend der mit der Längenmesseinrichtung gemessenen Länge des Kindes.

2. Pädiatrisches elektronisches Gerät nach Anspruch 1, **dadurch gekennzeichnet, dass** die Parametergruppen ausgewählt sind aus physischen Messungen, Normwerten von klinischen Parametern, Einstellungen für Ventilationsgeräte, Arzneimittel-Infusionsmengen, biochemischen Normwerten, Normwerten bezüglich Röntgenstrahlen und Elektrokardiogrammen, Infusions-Gerätegrößen, Luftweg-Gerätegrößen und Arzneimittelmengen für Wiederbelebung.

3. Pädiatrisches elektronisches Gerät nach Anspruch 2, **dadurch gekennzeichnet, dass** mindestens einige der speziellen Werte der verschiedenen Parameter für jede Länge in einer Datenbank gespeichert sind.

4. Pädiatrisches elektronisches Gerät nach Anspruch 1 oder 2, **dadurch gekennzeichnet, dass** die speziellen Werte einige der verschiedenen Parameter für jede Länge gemäß einer in der Speichereinrichtung gespeicherten Formel berechnet werden.

5. Pädiatrisches elektronisches Gerät nach einem der vorangehenden Ansprüche, **dadurch gekennzeichnet, dass** die Speichereinrichtung eine gemessene Länge eines Kindes speichert, bis sie zurückgesetzt wird.

6. Pädiatrisches elektronisches Gerät nach einem der vorangehenden Ansprüche, **dadurch gekennzeichnet, dass** die Messeinrichtung ein Band (14) aufweist, das in dem Gehäuse untergebracht und aus diesem herausziehbar ist, mit einer elektronisch betätigbaren Ableseeinrichtung (29) zum Ablesen des Ausmaßes, in welchem das Band aus dem Gehäuse herausgezogen worden ist.

7. Pädiatrisches elektronisches Gerät nach Anspruch 6, **dadurch gekennzeichnet, dass** es ein Rückziehmittel (22) zum automatischen Zurückziehen des Bandes in das Gehäuse aufweist.

8. Pädiatrisches elektronisches Gerät nach Anspruch 6 oder 7, **dadurch gekennzeichnet, dass** es ein Blockiermittel (24) aufweist zum Blockieren des Maßbandes, wenn es um einen gewünschten Betrag herausgezogen ist.

9. Pädiatrisches elektronisches Gerät nach einem der Ansprüche 6 bis 8, **dadurch gekennzeichnet, dass** die Speichereinrichtung zurückgesetzt wird, wenn das Maßband aus dem Gehäuse herausgezogen wird, nachdem es in das Gehäuse zurückgezogen worden ist.

10. Pädiatrisches elektronisches Gerät nach einem der Ansprüche 6 bis 9, **dadurch gekennzeichnet, dass** die Ablesevorrichtung einen optischen Sensor (29) auf weist, der auf Markierungen auf dem Band anspricht, wobei die Anzahl der an dem Sensor vorbeilaufenden Markierungen gezählt wird.

11. Pädiatrisches elektronisches Gerät nach Anspruch 10, bei dem die Markierung in Abschnitten angeordnete Öffnungen (28) in dem Band sind.

12. Pädiatrisches elektronisches Gerät nach einem der vorangehenden Ansprüche, bei dem die Gruppenwählmittel eine Anzahl von Knöpfen (30, 32, 34) umfassen, die von dem Benutzer des Gerätes wahlweise gedrückt werden, um auszuwählen, welche Gruppe von Parametern angezeigt werden soll.

13. Pädiatrisches elektronisches Gerät nach einem der vorangehenden Ansprüche, **dadurch gekennzeichnet, dass** sie eine Stromversorgung (58) und eine Notstromversorgung aufweist.

14. Pädiatrisches elektronisches Gerät nach Anspruch 13, **dadurch gekennzeichnet, dass** es eine "Sleep"-Funktion hat, durch die der Stromverbrauch herabgesetzt wird, wenn das Gerät in einem bestimmten Zeitraum nicht betätigt wird.

15. Pädiatrisches elektronisches Gerät nach Anspruch 13 oder 14, **dadurch gekennzeichnet, dass** es einen Niederspannungs-Monitor (60) zusammen mit einer Niederspannungs-Warnanzeige (20) aufweist.

16. Pädiatrisches elektronisches Gerät nach einem der vorangehenden Ansprüche, **dadurch gekennzeichnet, dass** das Anzeigemittel eine rückseitig beleuchtete LCD-Anzeigematrix mit geeigneten Treibern (20) aufweist.

## Revendications

1. Dispositif électronique pédiatrique (10) qui comprend :
un boîtier (12) ;
un moyen de mesure de longueur (14, 29) pour mesurer la longueur d'un jeune enfant ;
un moyen de mémoire (66) pour mémoriser des données relatives à divers paramètres dépendant du poids ; et
un moyen d'affichage (20) pour visualiser les valeurs des paramètres, **caractérisé en ce que** les paramètres sont regroupés en divers groupes ; et
le dispositif comprend en outre un moyen de sélection de groupe (30, 32, 34) pour sélectionner un groupe désiré de paramètres et un moyen de traitement (56) pour déterminer la valeur de divers paramètres dans le groupe sélectionné en fonction de la longueur du jeune enfant, telle que mesurée par les moyens de mesure de longueur.

2. Dispositif électronique pédiatrique selon la revendication 1, **caractérisé en ce que** les groupes de paramètres sont sélectionnés à partir de mesures physiques, de valeurs normales de paramètres cliniques, de réglages pour des équipements respiratoires, des quantités d'infusion de médicaments, des valeurs normales de biochimie, des valeurs normales relatives à des radiographies et des électrocardiogrammes, des tailles d'équipements d'infusion, des tailles d'équipements de voies respiratoires et des quantités de médicaments de réanimation.

3. Dispositif électronique pédiatrique selon la revendication 1 ou 2, **caractérisé en ce que** au moins certaines des valeurs particulières des divers paramètres pour chaque longueur sont mémorisées dans une base de données.

4. Dispositif électronique pédiatrique selon la revendication 1 ou 2, **caractérisé en ce que** les valeurs particulières de certains des divers paramètres pour chaque longueur sont calculés en fonction d'une formule, mémorisée dans le moyen de mémoire.

5. Dispositif électronique pédiatrique selon l'une quelconque des revendications précédentes, **caractérisé en ce que** le moyen de mémoire mémorise une longueur d'un jeune enfant qui a été mesurée, jusqu'à une réinitialisation.

6. Dispositif électronique pédiatrique selon l'une quelconque des revendications précédentes, **caractérisé en ce que** le moyen de mesure comprend une longueur de bande (14) logée dans le boîtier et extractible de celui-ci, avec un moyen de contrôle actionnable électroniquement (29) pour contrôler l'étendue à laquelle la bande a été extraite du boîtier.

7. Dispositif électronique pédiatrique selon la revendication 6, **caractérisé en ce qu'**il comprend un moyen de rétraction (22) pour rétracter automatiquement la bande dans le boîtier.

8. Dispositif électronique pédiatrique selon la revendication 6 ou 7, **caractérisé en ce qu'**il comprend un moyen de blocage (24) pour bloquer la bande de mesure quand elle a été extraite à une étendue désirée.

9. Dispositif électronique pédiatrique selon l'une quelconque des revendications 6 à 8, **caractérisé en ce que** le moyen de mémoire est réinitialisé quand la bande de mesure est extraite du boîtier après avoir été rétractée dans le boîtier.

10. Dispositif électronique pédiatrique selon l'une quelconque des revendications 6 à 9, **caractérisé en ce que** le moyen de contrôle comprend un capteur optique (29) sensible à des formations sur la bande, le nombre de formations passant au-delà du capteur étant compté.

11. Dispositif électronique pédiatrique selon la revendication 10, **caractérisé en ce que** les formations sont des ouvertures (28) à intervalles espacés dans la bande.

12. Dispositif électronique pédiatrique selon l'une quelconque des revendications précédentes, **caractérisé en ce que** le moyen de sélection de groupe comprend une pluralité de boutons (30, 32, 34) qui sont sélectivement actionnés par la personne utilisant le dispositif pour sélectionner quel groupe de paramètres doit être visualisé.

13. Dispositif électronique pédiatrique selon l'une quelconque des revendications précédentes, **caractérisé en ce qu'**il comprend une alimentation électrique (58) et une alimentation électrique de secours.

14. Dispositif électronique pédiatrique selon la revendication 13, **caractérisé en ce qu'**il comprend une fonction de « sommeil » moyennant quoi la consommation d'énergie est réduite si le dispositif n'est pas utilisé pendant une période de temps spécifiée.

15. Dispositif électronique pédiatrique selon la revendication 13 ou 14, **caractérisé en ce qu'**il comprend un moniteur à basse tension (60) conjointement à un annonciateur d'avertissement à basse tension (20).

16. Dispositif électronique pédiatrique selon l'une quelconque des revendications précédentes, **caractérisé en ce que** le moyen d'affichage comprend une matrice d'affichage à cristaux liquides à rétroéclairage avec gestionnaires appropriés (20).
